# EUROPEAN PATENT SPECIFICATION

(11) **EP 0 680 739 B1**
(45) Date of publication and mention of the grant of the patent: **17.03.1999**
(21) Application number: 95106571.3
(22) Date of filing: 02.05.1995
(51) Int. Cl.: A61F 13/15

(54) **Anatomically shaped sanitary napkin**
Anatomisch geformte Damenbinde
Serviette hygiénique de forme anatomique

(30) Priority: 03.05.1994 US 237073
(43) Date of publication of application: 08.11.1995
(73) Proprietor: McNEIL-PPC, INC., Milltown New Jersey 08850 (US)
(72) Inventor: Foley, Theodore, NJ 08816 (US); Worringer, Jennifer R., NJ 08520 (US); Grunhaus, Levi, NJ 08904 (US)
(74) Representative: Groening, Hans Wilhelm, Dipl.-Ing.

(56) References cited:
- EP-A- 0 238 053
- EP-A- 0 354 196
- EP-A- 0 525 778
- GB-A- 2 170 108

## Description

### Background of the Invention

This invention relates to an absorbent product, especially a sanitary napkin in accordance with the preamble of claims 1 and 10, configured to interact minimally with the distorting and compressing mass and pressures of the inside of the wearer's thighs. EP-A-0 525 778 is related to an absorbent product for use in absorbing human exudates, comprising a liquid permeable, body-facing cover, a liquid impermeable backing and an absorbent pad disposed therebetween. Said absorbent pad comprising a top, body-facing portion and a bottom, garment-facing portion having a center and transverse ends. Said top portion being generally rectangular. Said bottom portion being configured to be narrower in width at its center than at its transverse ends.

It is widely believed that the performance of an absorbent product, such as a sanitary napkin, can be enhanced by improving the contact of the napkin with the wearer's perineal area. This contact with the perineum can be broadly described as "product fit." Interaction between the napkin and the inside of the wearer's thighs can cause the napkin to move when the wearer walks, thus reducing product fit.

The lateral compression forces applied by the wearer's thighs can also cause the product to "collapse," thereby providing a smaller target zone for deposition of fluid and providing less coverage of the undergarment. Product collapse is more prevalent with thick, or full size, sanitary napkins than it is with thin or ultrathin products. It is believed that this is the case because there is little room between the inside of the thighs for a full thickness pad while there exists much more room further up from the inside of the thighs in the proximity of the perineal floor, which comprises the area between the "leg creases" or the point of juncture between the leg and torso. However, full size napkins are designed to be adhered to the inside of the wearer's underwear and, usually, there is a gap between the perineal floor and the underwear. Thus, there is often no other place for the napkin to be worn other than between the wearer's thighs. Thick napkins can also have comfort problems in addition to the performance problems described above. The movement of the napkin, its excess mass, and its abrasive interaction with the thighs can be a source of discomfort to the wearer.

Thin napkins have been developed and sold which tend to be more comfortable and tend to move less than thick napkins. Further, due to their thin profile, thin napkins are more discretely worn than full size napkins. Also, as stated above, thin napkins are not subject to the same problems of product collapse as full size napkins. However, many consumers still prefer the feeling of security they get from wearing thick napkins.

There is, therefore, a need for a discrete, thick, full protection sanitary napkin that provides the wearer with a feeling of security and is shaped to conform to the wearer's thighs, while still maximizing contact with the perineum, and minimizing product distortion caused by the pressures exerted by the wearer's thighs.

Some have tried to solve these problems by providing napkins in which the body-facing surface of the napkin is shaped to contact the user's body. Lassen et al., in U.S. 4,804,380, disclose a three-dimensionally shaped sanitary napkin which has been mechanically shaped by folding, molding or other techniques so that it has a raised portion located within the back one-half to two-thirds of the device that functions to cause the pad to readily fit and generally align itself with the wearer's anatomy.

U.S. 4,433,972 to Malfitano discloses a sanitary napkin in which the absorbent pad assembly comprises two pads, a relatively large pad of wood fluff or like absorbent material, and a substantially diamond shape second pad of lesser dimensions seated on said first pad and facing the wearer. The patent teaches that the diamond shaped portion of the pad engages the vaginal area of the user.

Others have tried to solve these problems by providing napkins with an essentially hourglass shape. U.S. 4,639,254 to LeGault et al. discloses a three-dimensionally hourglass-shaped sanitary napkin with a cellulose fluff insert that is contoured or outwardly bulging on the baffle side to create a thicker region near the center of the hourglass.

U.K. Patent Application GB 2 191 098A discloses hourglass shaped napkins in which the center portion of the body-facing surface of the pad is raised.

U.S. 4,770,657 to Ellis et al. discloses a curved elongate absorbent pad having a liquid impermeable backing, an upper reinforcing absorbent member, a reservoir absorbent member and a liquid permeable body-side member. The lower absorbent reservoir member is hourglass or dog-bone shape. The pad is preferably curved, by the inclusion of elastic, to conform to the wearer's body.

U.S. 4,687,478 to Van Tilburg discloses a sanitary napkin purported to provide panty coverage comprising an hourglass shaped absorbent having flaps extending from the edges which bend around and attach to the outer crotch portion of the wearer's undergarment.

U.S. 4,795,453 to Wolfe discloses a napkin having a "dogbone" shaped absorbent with a thicker middle bridge portion connecting planar end pieces. However, coverage is maintained by filling in the edges of the center portion of the "dogbone" with a soft and absorbent material. Although the edge material may be softer than the absorbent core of the pad, thigh interference would still be expected.

U.S. 4,631,062 to Lassen et al. discloses a labial pad having an anatomically conformable configuration with a generally ovate geometry. It is comprised of a fluid absorbent body in which the posterior region of the top, body-facing surface has raised profile for projection within the vestibule intermediate the labia majora.

U.S. 4,701,177 to Ellis et al. discloses a curved absorbent pad in which the absorbent member is shaped generally in a manner that provides a narrower middle portion of absorbent material. The middle portion is also preferably thicker than the absorbent portions at the ends of the pad. The pad is curved to conform to the pudendum.

U.S. 4,848,572 to Herrera discloses a sanitary napkin having a curved, trapezio-pyramidal shape closely complementary to the female anatomy. The napkin has a voluminous and thick orthocentral portion which tapers down to the sides and ends of the napkin.

U.S. 5,043,206 to Ternstrom discloses an absorption body intended for articles such as diapers, sanitary napkins and the like having a soft, T-shaped, first layer intended to be placed against the wearer's body and a second, heavily compressed layer having a width fitting the wearer's crotch.

U.S. 5,092,860 to Pigneul discloses a sanitary napkin characterized by the inclusion in its upper body contacting side of two series of discrete indentations. One series of indentations is positioned adjacent to one of the longitudinal edges of the absorbent core and the other series of indentations is positioned adjacent to the opposite longitudinal edge of the absorbent core.

### Summary of the Invention

This invention relates to a novel configuration for absorbent products in accordance with the characterizing portion of claims 1 and 10 which minimally interacts with the distorting and compressing mass and pressures of the inside of the user's thighs, thereby resulting in a comfortable napkin that covers the perineal area and fits the inner thigh space efficiently and without excess material. More specifically, this invention relates to an absorbent product for use in absorbing human exudates, comprising
a liquid permeable, body-facing cover;
a liquid impermeable backing; and
an absorbent pad disposed therebetween,
   said absorbent pad comprising a top, body-facing portion and a bottom, garment-facing portion; said top portion being generally rectangular; and said bottom portion being configured to be narrower in width, and preferably thicker at its center than at its transverse ends; said top portion of said absorbent pad is stabilized pulp. Such a product provides the non-interfering fit and non-collapsing benefits of a thin napkin while it can also provide the feeling of security and the absorbing benefits of a full thickness napkin.

This invention also relates to the specially configured absorbent pad of this invention which can be used in a variety of absorbent products.

### Brief Description of the Drawings

Figure 1 is a perspective view of a sanitary napkin of this invention, with the garment-facing surface of the napkin facing upwards.
Figures 2 and 3 are cross-sectional views of the sanitary napkin of Figure 1.

### Detailed Description of the Invention

This invention is susceptible of embodiment in many different forms. A preferred embodiment is shown in the drawings and is described in detail below. This disclosure is considered to be an example of the invention and is not intended as limiting of the scope of the invention.

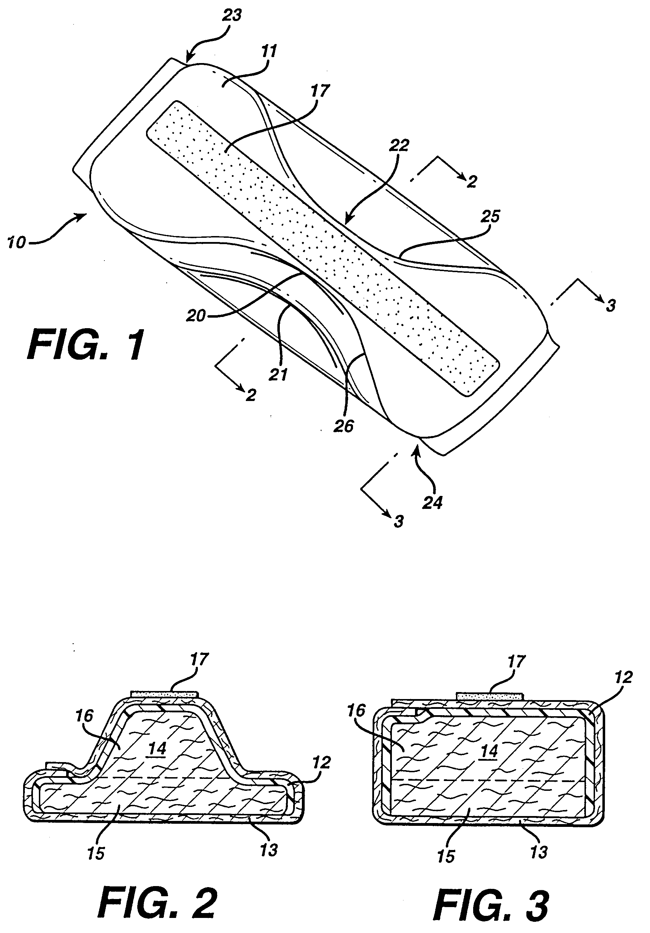

Figures 1, 2 and 3 illustrate how the bottom part of the absorbent pad in a sanitary napkin of this invention has been "carved out" to match the configuration of the thighs of the wearers. This design is new and unexpectedly different from the prior art. Previous inventions directed to fitting the perineal area focused on mirroring the contours of the perineal surface rather than the volume of the subperineal-interthign space.

Referring to the Figures, there is shown a sanitary napkin **10** of this invention, with the garment-facing surface **11** of the napkin facing upwards. The napkin comprises a liquid impermeable backing **12** and a liquid permeable cover **13**. As shown in the illustrated embodiment, the liquid permeable cover **13** is wrapped around the entire napkin; however, in another embodiment the liquid impermeable backing **12** could form the garment-facing surface of the product and the liquid permeable cover **13** could be provided on the body-facing surface only, on the body-facing surface and sides of the product, or on the body-facing surface, sides and a portion of the garment-facing surface.

Between the backing **12** and cover **13** is the absorbent pad **14** comprising a body-facing portion **15** and a garment-facing portion **16**. The body-facing portion **15** is generally rectangular. "Generally rectangular" means generally, but not necessarily rectangular, i.e., having transverse edges shorter than longitudinal edges. The garment-facing portion **16** is essentially "carved out" to be narrower in width at the longitudinal center of the pad **22** than at its transverse ends **23** and **24**.

The carved out portion of the pad can be either symmetric or non-symmetric from front to back (longitudinally).

As shown in Figure 1, the longitudinal edges **25** and **26** of the bottom, garment-facing portion of the absorbent pad are concave. Typical, preferred shapes for the carved-out bottom portion of the pad are hourglass and dog-bone shapes. While the carved out section could be in the form of a series of complex curves to exactly match the configuration of the inner thighs, it is more convenient with respect to mold and tooling design to produce the product by considering the shape to be a constant radius of curvature approximating the user's thighs. In one preferred embodiment, the radius of curvature at the upper edge of the curve, represented in Figure 1 by arc **20**, is slightly less than the radius of curvature at the lower edge of the curve, represented by arc **21**. This results in the garment-facing portion **16** tapering toward the body-facing portion **15**, rather than there being a straight edge. For example, the radius of curvature represented by **20** might be approximately five inches, and the radius of curvature at **21** might be approximately seven inches. Alternatively, the radius of curvature at **21** could also be five inches but the center point from which the radius is struck could be moved closer to the pad.

The thickness of the pad, that is the z-directional distance between the body-facing surface and the garment-facing surface, should preferably be sufficient to fill any space between the inside of the wearer's underwear and the perineum. Typically, this thickness should be from about 15,2 mm to about 50,8 mm 0.6 to 2 inches). Preferably, it should be from about 15,2 mm to about 27,9 mm (0.6 to 1.1 inches). Also, as stated above, in a preferred embodiment, the thickness of the pad should taper from the center section to the longitudinal and transverse ends to improve fit and to allow the product to be worn more discretely.

The absorbent pad **14** may be made from conventional ground wood pulp. Preferably, however, the body-facing portion **15** is stabilized, e.g., by compression of the pulp to a higher density or by inclusion of a material having higher dry or wet stability, or both. Examples of such materials are compressed sphagnum moss, compressed superabsorbent composite, or hydrophilic foams such as aminoether polymer foams or hypol polyurethane foam. Additionally, the body-facing portion **15** of the pad may be stabilized by inclusion of long staple length hydrophilic or hydrophobic fibers blended with the pulp or short staple length synthetic wood pulp. The staple fibers are preferably at least partially thermoplastic and the blend is thermally bonded for maximum stability against collapse when wetted.

The garment-facing, or sculpted, portion **16** of the absorbent pad may also be made of wood pulp or stabilized materials described above. Since it will tend to stay drier than the upper portion and will be subjected to less forces, it is not as important that the lower portion be stabilized. The absorbent pad may be a single, unitary structure or may comprise two pad portions adjacent to one another and optionally adhered with construction adhesives, etc., to form a unitary structure. The shaped portion of the absorbent pad may be formed from a pulp pad by molding and forming means known in the art, such as vacuum molding or die cutting. Such processes are described in U.S. Patent 5,004,579 (Wislinski et al.).

The liquid impermeable backing **12** will generally comprise a fluid impervious material such as polyethylene or polypropylene. As shown in the figures, the liquid impermeable layer is preferably formed to the same configuration as the lower portion of the absorbent pad. This can be accomplished by either thermal or mechanical means or a combination thereof, such as vacuum forming. Alternatively, however, the impermeable backing and optional cover overlaying the backing may loosely cover the garment-facing surface of the absorbent pad, not adhering to the carved-out shape of the pad.

The liquid permeable cover **13** will generally comprise a film or fabric having a high degree of moisture permeability. For example, the fabric may be comprised of fibrous material made from polyester, polyethylene, polypropylene, bicomponent fiber, nylon, rayon, or the like. The most suitable fabrics have unusually high elongation, loft, softness and drape characteristics. Films which are perforated or noncontinuous are also satisfactory. Though the cover is moisture permeable, it is preferably of the type which after permeation of the moisture, prevents strike-back of the body fluid when the absorbent structure is approaching saturation.

Attachment means **17** serve to securely adhere the product to the wearer's undergarment. The attachment means may comprise one or more adhesive lines covered with release strips (not shown) which, when peeled from the adhesive strips, leave the adhesive tacky. Alternatively, the attachment means may comprise pressure-sensitive adhesive tape, said tape having a first face permanently adhered to the garment-facing surface **11** of the product and an opposite second face adapted to be temporarily attached to the wearer's garment.

## Claims

1. An absorbent product for use in absorbing human exudates, comprising:
- a liquid permeable, body-facing cover (13),
- a liquid impermeable backing (12), and
- an absorbent pad (14) disposed therebetween, said absorbent pad comprising a top, body-facing portion (15) and a bottom, garment-facing portion (16) having a center and transverse ends (23, 24),
- said top portion (15) being generally rectangular, said bottom portion (16) being configured to be narrower in width at its center than at its transverse ends (23, 24), characterized in that said top portion (15) of said absorbent pad (14) is stabilized pulp.

2. An absorbent product of claim 1, wherein said bottom portion (16) is thicker at its center than at its transverse ends (23, 24).

3. An absorbent product of claim 1, wherein said bottom portion (16) has longitudinal edges (25, 26) which are concave.

4. An absorbent product of claim 1, wherein said bottom portion (16) of said absorbent pad (14) is hourglass-shaped.

5. An absorbent product of claim 1, wherein said bottom portion (16) of said absorbent pad (14) is dogbone-shaped.

6. An absorbent product of claim 1, wherein said bottom portion (16) of said absorbent pad (14) tapers into said top portion (15) of said absorbent pad (14).

7. An absorbent product of claim 1, wherein said absorbent pad (14) is a unitary strcture.

8. An absorbent product of claim 1, wherein said top and bottom portions (15, 16) of said absorbent pad (14) are separate and different.

9. An absorbent product of claim 1, which is a sanitary napkin (10).

10. An absorbent pad (22) having a body-facing surface, a garment-facing surface (11), a center and transverse ends (23, 24), wherein said body-facing surface is generally rectangular and wherein said garment-facing surface (11) is narrower in width at its center than at its transverse ends (23, 24), characterized in that the top portion (15) of said absorbent pad (22) is stabilized pulp.

11. An absorbent pad of claim 10, wherein the pad (22) is thicker at its center than at its transverse ends (23, 24).

12. An absorbent pad of claim 10, wherein said bottom portion (16) has longitudinal edges (25, 26) which are concave.

13. An absorbent pad of claim 10, wherein said bottom portion (16) of said absorbent pad (22) is hourglass-shaped.

14. An absorbent pad of claim 10, wherein said bottom portion (16) of said absorbent pad (22) is dogbone-shaped.

15. An absorbent pad of claim 10, wherein said bottom portion (16) of said absorbent pad (22) tapers into said top portion (15) of said absorbent pad (22).

16. An absorbent pad of claim 10, wherein said absorbent pad (22) is a unitary structure.

17. An absorbent pad of claim 10, wherein said top and bottom portions (15, 16) are separate and different.

## Patentansprüche

1. Absorptionserzeugnis zum Absorbieren menschlicher Ausscheidungen, umfassend:
- eine flüssigkeitsdurchlässige, dem Körper zugewandte Abdeckung (13),
- eine flüssigkeitsundurchlässige Rückschicht (12), und
- ein Absorptionskissen (14), das dazwischen angeordnet ist, wobei das Absorptionskissen einen oberen, dem Körper zugekehrten Teil (15) und einen unteren, der Bekleidung zugekehrten Teil (16) mit einer Mitte und querverlaufenden Enden (23, 24) umfaßt,
- der Oberteil (15) im allgemeinen rechteckig ist und der Unterteil (16) in seiner Mitte schmaler als seine Querenden (23, 24) ist, dadurch gekennzeichnet, daß der Oberteil (15) des Absorptionskissens (14) stabilisierte Pulpe ist.

2. Absorptionserzeugnis nach Anspruch 1, wobei der Unterteil (16) in seiner Mitte dicker als seine Querenden (23, 24) ist.

3. Absorptionserzeugnis nach Anspruch 1, wobei der Unterteil (16) Längskanten (25, 26) hat, die konkav sind.

4. Absorptionserzeugnis nach Anspruch 1, wobei der Unterteil (16) des Absorptionskissens (14) stundenglasförmig ist.

5. Absorptionserzeugnis nach Anspruch 1, wobei der Unterteil (16) des Absorptionskissens (14) sanduhrförmig ist.

6. Absorptionserzeugnis nach Anspruch 1, wobei der Unterteil (16) des Absorptionskissens (14) zum Oberteil (15) des Absorptionskissens (14) hin verjüngt ist.

7. Absorptionserzeugnis nach Anspruch 1, wobei das Absorptionskissen (14) eine einheitliche Struktur aufweist.

8. Absorptionserzeugnis nach Anspruch 1, wobei die Ober- und Unterteile (15, 16) des Absorptionskissens (14) getrennt und unterschiedlich sind.

9. Absorptionserzeugnis nach Anspruch 1, welches eine hygienische Binde (10) ist.

10. Absorptionskissen (22) mit einer dem Körper zugewandten Seite, einer der Bekleidung zugekehrten Seite (11), einer Mitte und Querenden (23, 24), wobei die dem Körper zugekehrte Seite im allgemeinen rechtwinklig ist und wobei die der Bekleidung zugekehrte Seite (11) in ihrer Mitte schmaler als an ihren Querenden (23, 24) ist, dadurch gekennzeich-net, daß der Oberteil (15) des Absorptionskissens (22) stabilisierte Pulpe ist.

11. Absorptionskissen nach Anspruch 10, wobei das Kissen (22) in der Mitte dicker als an seinen Querenden (23, 24) ist.

12. Absorptionskissen nach Anspruch 10, wobei der Unterteil (16) Längskanten (25, 26) hat, die konkav sind.

13. Absorptionskissen nach Anspruch 10, wobei der Unterteil (16) des Absoptionskissens (22) stundenglasförmig ist.

14. Absorptionskissen nach Ansprhuch 10, wobei der Unterteil (16) des Absorptionskissens (22) sanduhrförmig ist.

15. Absorptionskissen nach Anspruch 10, wobei der Unterteil (16) des Absorptionskissens (22) zum Oberteil (15) des Absorptionskissens (22) hin verjüngt ist.

16. Absorptionskkissen nach Anspruch 10, wobei das Absorptionskissen eine einheitliche Struktur aufweist.

17. Absorptionskissen nach Anspruch 10, wobei die Ober- und Unterteile (15, 16) getrennt und unterschiedlich sind.

## Revendications

1. Un produit absorbant destiné à être utilisé pour absorber les exsudats d'origine humains, comprenant :
- une feuille de dessus perméable aux liquides et faisant face au corps (13);
- une feuille de dessous faisant face aux vêtements (14); et
- un molleton absorbant (14) disposé entre ces deux feuilles, ledit molleton absorbant comprenant une partie de dessus faisant face au corps (15) et une partie de dessous faisant face aux vêtements (16) munies d'une partie centrale et d'extrémités transversales (23, 24);
- ladite partie de dessus (15) étant généralement rectangulaire, ladite partie de dessous (16) étant configurée de telle sorte qu'elle soit plus étroite en largeur au niveau de son centre qu'au niveau de ses extrémités transversales (23, 24), caractérisée en ce que ladite partie de dessus (15) dudit molleton absorbant (14) est de la pulpe stabilisée.

2. Un produit absorbant selon la revendication 1, dans lequel ladite partie de dessous (16) est plus épaisse au niveau de son centre qu'au niveau de ses extrémités transversales (23, 24).

3. Un produit absorbant selon la revendication 1, dans lequel ladite partie de dessous (16) a des bords longitudinaux (25, 26) qui sont concaves.

4. Un produit absorbant selon la revendication 1, dans lequel ladite partie de dessous (16) dudit molleton absorbant (14) a la forme d'un sablier.

5. Un produit absorbant selon la revendication 1, dans lequel ladite partie de dessous (16) dudit molleton absorbant (14) a la forme d'un os pour chien.

6. Un produit absorbant selon la revendication 1, dans lequel ladite partie de dessous (16) dudit molleton absorbant (14) s'effile dans ladite partie de dessus (15) dudit molleton absorbant (14).

7. Un produit absorbant selon la revendication 1, dans lequel ledit molleton absorbant (14) est une structure unitaire.

8. Un produit absorbant selon la revendication 1, dans lequel lesdites parties de dessus et de dessous (15, 16) dudit molleton absorbant (14) sont séparées et différentes.

9. Un produit absorbant selon la revendication 1, qui est une serviette hygiénique (10).

10. Un molleton absorbant (22) ayant une surface faisant face au corps, une surface faisant face aux vêtements (11), une partie centrale et des extrémités transversales (23, 24), dans lequel ladite surface faisant face au corps est généralement rectangulaire et dans lequel ladite surface faisant face aux vêtements (11) est plus étroite en largeur au niveau de son centre qu'au niveau de ses extrémités transversales (23, 24), caractérisé en ce que la partie de dessus (15) dudit molleton absorbant (22) est de la pulpe de bois stabilisée.

11. Un molleton absorbant selon la revendication 10, dans lequel le molleton (22) est plus épais au niveau de son centre qu'au niveau de ses extrémités transversales (23, 24).

12. Un molleton absorbant selon la revendication 10, dans lequel ladite partie de dessous (16) a des bords longitudinaux (25, 25) qui sont concaves.

13. Un molleton absorbant selon la revendication 10, dans lequel ladite partie de dessous (16) dudit molleton absorbant (22) a la forme d'un sablier.

14. Un molleton absorbant selon la revendication 10, dans lequel ladite partie de dessous (16) dudit molleton absorbant (22) a la forme d'un os pour chien.

15. Un molleton absorbant selon la revendication 10, dans lequel ladite partie de dessous (16) dudit molleton absorbant (22) s'effile dans ladite partie de dessus (15) dudit molleton absorbant (22).

16. Un molleton absorbant selon la revendication 10, dans lequel ledit molleton absorbant (22) est une structure unitaire.

17. Un molleton absorbant selon la revendication 10, dans lequel les parties de dessus et de dessous (15, 16) sont séparées et différentes.
